Europäisches Patentamt

**European Patent Office**

**Office européen des brevets**

(19)

(11) Publication number: **0 074 819**
**A2**

(12) # EUROPEAN PATENT APPLICATION

(21) Application number: **82304779.0**

(22) Date of filing: **10.09.82**

(51) Int. Cl.³: **A 61 K 7/06**

(30) Priority: **14.09.81 US 302100**
**06.07.82 US 395502**

(43) Date of publication of application:
**23.03.83 Bulletin 83/12**

(84) Designated Contracting States:
**AT BE CH DE FR GB LI LU NL**

(71) Applicant: **Amway Corporation**
**7575 East Fulton Raod**
**Ada Michigan(US)**

(72) Inventor: **Glover, David Alan**
**2119 Engleside**
**South East Kentwood Michigan(US)**

(74) Representative: **Robinson, Anthony John**
**Metcalf et al,**
**Kilburn & Strode 30 John Street**
**London, WC1N 2DD(GB)**

(54) Anti-dandruff cream rinse conditioner.

(57) A water base anti-dandruff cream rinse conditioner incorporating zinc pyrithione includes a cationic polymer and a suspension system consisting of (1) one of glucan gum, guar gum or mixtures thereof, and (2) hydroxyethylcellulose, as well as other optional conditioning agents and ingredients.

EP 0 074 819 A2

1.

# ANTI-DANDRUFF CREAM RINSE CONDITIONER

The present invention relates to anti-dandruff compositions. Anti-dandruff shampoos have been sold in a variety of different formulations for a number of years. Many different types of anti-dandruff agents are employed. Insoluble particulate zinc pyrithione (or zinc pyridinethione) (zinc bis (2-pyridylthio)-n-oxide) has perhaps the most widely acknowledge efficacy as an anti-dandruff agent.

It has been suggested that cationic polymers can increase the efficacy of zinc pyrithione in anti-dandruff shampoos by enhancing the deposition and retention of the water insoluble zinc pyrithione particles on the hair and scalp. United States Patent 3,489,686 suggests that polyethylenimine or the reaction product of polyethylenimine and ethylene oxide or propylene oxide performs such a function. United States Patent 3,580,853 teaches that a cationic homopolymer comprising a particular cellulose derivative achieves this objective. United States Patent 3,761,417 suggests a piperidinium chloride for this purpose.

It has also been suggested in the literature that an acrylamide copolymer of dimethyldiallyl ammonium chloride of a molecular weight of around 500,000, known by the trade name Merquat 550 and available from Merck & Co., Inc., might be useful in shampoos because it is said to contribute to detangling and improve wet and dry compatability without a greasy feeling. This acrylamide copolymer is said to impart richness and lubricity to the shampoo foam. This chemical is known in the art as Quaternium 41, per the C.T.F.A. Cosmetic Ingredient Dictionary, Second Edition.

Since this particular polymer is also cationic in nature, we thought to take advantage of both its shampoo improving qualities and its cationic properties, even though. it is different from the cationic polymers cited in the patents above, to create a more desirable, and more efficacious zinc pyrithione anti-dandruff shampoo. Unfortunately, this combination was unsuccessful. We were unable to produce a commercially acceptable shampoo based on this acrylamide copolymer and zinc pyrithione.

The resulting shampoos were unstable and/or tended to be slimy. Indeed, we know of no commercial anti-dandruff shampoo based on this acrylamide copolymer, even though such use has been suggested: A cellulose thickener sold as Avicel GLG11, Quaternium 41 and zinc pyrithione were stable initially in a shampoo, but were granular, thick and not stable over a long period of time. Hydroxypropylmethylcellulose thickener (Methocel), Quaternium 41 and zinc pyrithione were initially stable, but not over long periods of time, unless excessive amounts of Methocel were used. Increasing Methocel amounts increases sliminess.

In addition to a large number of anti-dandruff shampoos, at least one anti-dandruff rinse has been marketed commercially. It did not utilize zinc pyrithione as its active anti-dandruff ingredient. Nor did it incorporate any conditioners of the type typically employed in cream rinse conditioners. We are unaware of any anti-dandruff rinse conditioners currently on the market.

Part of the problem in creating an anti-dandruff rinse conditioner is that some conditioners are cationic polymers, which seem to be incompatible with zinc pyrithione, particularly suspensions thereof. Beecham Group Ltd. claims in European Patent Publication EPC-A-7704 that this can be overcome in a rinse conditioner by suspending the zinc pyrithione in a cationic polymer such as polymer JR, which is Quaternium 19 in the C.T.F.A. Cosmetic Ingredient Dictionary. We were unable to verify the results claimed by this patent publication, at least in any commercially acceptable embodiment.

Indeed from the paucity of anti-dandruff rinses and the total absence of anti-dandruff cream rinse conditioners available on the market, it appears that attempts to create anti-dandruff rinses and especially anti-dandruff rinse conditioners have generally been frustrated. Based upon our own work, we believe that attempts to create anti-dandruff rinses based on zinc pyrithione, which rinses also employ cationic conditioning polymers, have been frustrated due to the incompatibility of the cationic polymeric materials with zinc pyrithione and/or suspending agents used to keep the water insoluble zinc pyrithione in suspension.

Surprisingly, we have found that the problems can be overcome and an anti-dandruff cream rinse conditioner comprising zinc pyrithione and a cationic polymer can be made using, in accordance with the present invention, hydroxyethylcellulose and either glucan gum or guar gum (or mixtures thereof) as the suspending ingredients. Yet, this combination was not effective in a shampoo formulation.

Indeed to our surprise, we discovered that even though the cationic acrylamide copolymer discussed above (Quaternium 41) did not work to commercial satisfaction in a zinc pyrithione shampoo, the aforesaid cationic acrylamide copolymer and zinc pyrithione do work exceptionally compatibly as an effective anti-dandruff conditioner rinse combination in the presence of glucan gum, guar gum or mixtures thereof and hydroxyethylcellulose. Indeed, the resulting anti-dandruff conditioner rinse shows surprisingly exceptional efficacy as an anti-dandruff composition. While anti-dandruff shampoos seem to require 2% active zinc pyrithione, the anti-dandruff cream rinse conditioner of the present invention was shown to be equally efficacious with only 1% active zinc pyrithione, though obviously the amounts may vary.

According to another aspect of the invention, a water base, anti-dandruff rinse conditioner is characterised by the use of the combination of an effective amount of zinc pyrithione and a cationic acrylamide copolymer of dimethyldiallyl ammonium chloride having a molecular weight of approximately 500,000

The objects, advantages and features of the composition of the present invention will be more fully understood and appreciated by reference to the following more detailed description of preferred features of the invention.

In the preferred anti-dandruff, cream rinse conditioners, zinc pyrithione in an amount sufficient to act as an effective anti-dandruff agent is mixed with from about .25 to about 5.0% of a cationic polymer in sufficient (1) glucan gum, guar gum or both and (2) hydroxyethylcellulose to keep these active ingredients in a smooth, consistent suspension.

5.

Up to as much as 18% of other conditioning agents are also most preferably used to give the product conventional cream rinse properties. Miscellaneous additives may also be used such as jojoba oil, perfume and the like. All percentages herein are by weight unless otherwise indicated.

The most preferred cationic polymer is identified in the C.T.F.A. Cosmetic Dictionary as Quaternium 41. It is an acrylamide copolymer of dimethyldiallyl ammonium chloride and has a molecular weight of approximately 500,000. It gives the most markedly preferred commercial product. This is particularly surprising in that we were unable to formulate this cationic polymer into an anti-dandruff shampoo formulation using zinc pyrithione. Shampoo formulations based on this cationic polymer were granular and thick and not stable over a long period of time.

The cationic polymer serves not only as a conditioner, but also enhances the substantivity of the zinc pyrithione. It does this, it is believed, by enhancing the attraction of zinc pyrithione particles for the scalp and hair. While the range of cationic polymer is from about .25 to about 5% by weight, approximately 1% is most preferable.

Other cationic polymers can be substituted for Quaternium 41, though they are less preferred and do not yield as cosmetically attractive commercial preparation. The following are alternative cationic polymers which we have found effective in place of Quaternium 41 and which are readily compatible in suspension with zinc pyrithione, in the presence of glucan gum or guar gum or mixtures thereof, and hydroxyethylcellulose:

Quaternium 22, which is v-gluconamidopropyl dimethyl-2-hydroxyethyl ammonium chloride;

Quaternium 23, which is quaternary ammonium polymer formed by the reaction of dimethyl sulphate and a copolymer of vinyl pyrrolidone and dimethyl aminoethylmethacrylate;

Quaternium 26, which is mink-amidopropyl di-methyl-2-hydroxyethyl ammonium chloride;

Quaternium 40, which is a highly charged cationic dimethyldiallyl ammonium chloride homopolymer;

Quaternium 19, which is a polymer of hydroxyethyl cellulose reacted with epichlorohydrin and then quaternized with trimethyl amine; and

Polyquaternium 3, which is a copolymer of trimethylammonium ethyl methacrylyte methosulphate and acrylimide.

It is obvious, to one skilled in the art, that alternative polymers that may be substituted are numerous. Quaternium 33 and Quaternium 31, for example, are likely candidates for use as the cationic polymer. The names used are, as in the case of Quaternium 41, found in the C.T.F.A. Cosmetic Ingredient Dictionary, Second Edition.

Zinc pyrithione must be employed in the finished product at such a level as to provide effective, clinically demonstrable, dandruff control. Prior workers have stated that anywhere from .1 to 10% by weight zinc pyrithione should be used. As a practical matter, probably more that 5% would be redundant and, in most shampoos, approximately 2% active zinc pyrithione is optimum. Thus the preferred embodiment employs from about .25% to about 5% by weight active zinc pyrithione. Most preferably, about 1% active zinc pyrithione is used. The word "active" refers to the fact that zinc pyrithione usually comes in aqueous dispersion such that the level of "active" zinc pyrithione will be somewhat less than the percentage of the total dispersion added to the formulation. Hence 2.1% by weight of a 48% zinc pyrithione dispersion would actually constitute 1% active zinc pyrithione by weight.

The suspension system for the product of the preferred embodiment comprises a combination of (1) hydroxyethylcellulose and (2) glucan gum, or guar gum or mixtures thereof. This combination is the only one found which works commercially satisfactorily. This is particularly surprising in view of the fact that this combination was not effective in suspending zinc pyrithione and a cationic polymer in a shampoo formulation.

In this regard, it will of course be appreciated that by using excessive amounts of conventionally known suspending agents, almost any such conventional suspension agent will keep zinc pyrithione in suspension in the presence of a cationic polymer. However as a practical commercial matter, such products would be far too thick, or would lose effectiveness due to flocculation of the cationic polymer which may be masked by the thickness of the product, or would be too expensive or a combination of these. By using the combination of hydroxyethylcellulose and glucan gum or guar gum or a mixture of both, one can control both the viscosity and aesthetic qualities of the cream rinse conditioner very accurately.

Preferably, the suspension combination comprises from 25 to 75% hydroxyethylcellulose and conversely from 75 to 25% of the gum. Most preferably, the two ingredients are employed in equal proportions by weight. From about .5 to about 5% of the suspension combination is utilized in the product, and most preferably about 1%. Hence it is most preferable that .5% glucan gum or guar gum or a mixture thereof and .5% hydroxyethyl-cellulose are used in the final product.

Glucan and guar gum are high molecular weight, linear, nonionic polysaccharides. While they can be modified to be made cationic or anionic, or to be made nonlinear, such modifications are not desirable in the context of the present invention.

The glucan gum and guar gum used must be of a refined grade. Thus it has been found that a refined grade of glucan gum in powder form with a white to cream clear appearance, and having 90% glucan content and only 3% ash and .4% nitrogen works very effectively in the present invention. On the other hand, so-called industrial quality glucan gum which contains only 65% glucan is not operable. This compound is a more cream colour powder, has 4% ash and 2.5% nitrogen. In a 1% solution, the industrial quality glucan gum shows a viscosity at 30 rpm Brookfield LVF viscometer, Spindle No. 3, of 1,000 to 1,200, whereas the refined grade has a viscosity of 2,100 to 2,400. Similarly, the guar gum used should be a similarly highly purified or refined powder. It's viscosity at 25$^{\circ}$C. on the Brook-field viscometer, Model RVF, in a 1% solution should be approximately 4,500 cps.

Other conditioning ingredients or agents can be employed in the cream rinse formulation, in quantities which preferably do not exceed 18%.

**0074819**

Most preferably, approximately 9% of other conditioners are employed. One such conditioner is a commercially available mixture of stearyl alcohol and cetrimonium bromide. This product comprises about 75% stearyl alcohol and 25% cetrimonium bromide. From 1 to 5% of this ingredient, and preferably around 3% of this ingredient is employed. Up to 4%, and preferably about 2% steartrimonium hydrolyzed animal protein is also utilized. Up to 8% and preferably about 4% isostearamido propyl morpholine lactate is additionally employed as a conditioning agent in the most preferred embodiment.

Other conventional conditioning agents can be substituted into the formula. Thus we have employed, as alternatives to stearyl alcohol and cetrimonium bromide, stearalkonium chloride, tallow amidoethyl polyhydroxy ether ammonium chloride, hydrogenated tallow polyoxyethylene ammonium ethosulphate, cetrimonium chloride and dialkyl dimethyl ammonium chloride, and other quaternary ammonium salts typically used in cream rinse conditioners. These are all known cream rinse conditioning agents and it is believed that their interchangeability as well as the possibility of using numberous substitutes and the reasons for using one or more are well-known in the art.

Items such a jojoba oil and perfume are optional in the formula. It is preferred that deionized water be used in formulating the composition. Other ingredients will undoubtedly occur to those skilled in the art for possible inclusion in a product made in accordance with the present invention.

The following is believed to be the most preferred formulation for a commercial product:

| MATERIAL | % BY WEIGHT |
| --- | --- |
| Deionized water | 86.0 |
| Hydroxyethylcellulose | 0.5 |
| Glucan gum or guar gum or a mixture thereof | 0.5 |
| Steartrimonium Hydrolyzed Animal Protein | 2.0 |
| Stearyl Alcohol and Cetrimonium Bromide (75/25) | 3.0 |
| Isostearamido Propyl Morpholine Lactate | 4.0 |
| Quaternium 41 | 1.0 |
| Jojoba Oil | 0.5 |
| Zinc Pyrithione (48% dispersion) | 2.1 (1% active) |
| Perfume | 0.4 |
| Total | 100.0% |

The resulting formulation is smooth, consistent and stable. Accelerated aging tests have determined the product to be very stable, providing a uniform drug (active) dosage to the consumer even with aging. One does not even have to include an instruction to shake this composition before using to ensure homogeneity. It appears to be very stable (homogeneous) for long periods of time, requiring no expiration date on the container.

The conclusions reached in connection with the present invention are supported by extensive experimental data. The following tables summarize that data (with some of the experiments reported actually being based on more than one attempt). In all cases, the quantities of zinc pyrithione used were 1 to 2% active. The quantities of all other ingredients expressed in these tables were from .5 to2%, usually 1%.

## TABLE I

### FORMULATIONS IN ACCORDANCE WITH
### THE PRESENT INVENTION

| MIXTURE | RESULT |
|---------|--------|
| Quaternium 41, hydroxyethyl-cellulose, glucan gum and zinc pyrithione | very stable composition, very smooth, with less suspension agent required than to achieve any kind of stability with any other suspending agents. Better long term stability than with other suspending agent. |
| Quaternium 22, Hydroxyethyl-cellulose, glucan gum and zinc pyrithione | Same as above, except that the product did not have as nice an aesthetic, cosmetic appearance as when Quaternium 41 was employed above. |
| Quaternium 40, hydroxyethyl-cellulose, glucan gum and zinc pyrithione | About the same as above when Quaternium 22 was used. |
| Quaternium 26, hydroxyethyl-cellulose, glucan gum and zinc pyrithione | About the same as above when Quaternium 22 was used. |
| Quaternium 23, hydroxyethyl-cellulose, glucan gum and zinc pyrithione | About the same as above when Quaternium 22 was used. |
| Polyquaternium 3, hydroxy-ethylcellulose, glucan gum and zinc pyrithione | Abount the same as above when Quaternium 22 was used. |
| Polyquaternium 19, hydroxy-ethylcellulose, glucan gum and zinc pyrithione | About the same as above when Quaternium 22 was used. |
| Quaternium 41, hydroxyethyl-cellulose, guar gum and zinc pyrithione | Very smooth, clean, and has good stability. Very comparable to the first example above. |

The last test actually reflects work with three
different commercially available guar gums.  All
operated comparably in conjunction with hydroxyethylcellulose to give the results reported.

## TABLE II

### FORMULATIONS USING QUATERNIUM 41, ZINC PYRITHIONE AND OTHER SUSPENSION AGENTS

| MIXTURE | RESULT |
|---|---|
| Cellulose thickener (trademark product Avicel GLG11), Quaternium 41 and zinc pyrithione | Inconsistent, sometimes granular, sometimes just barely acceptable, at least initially, though product tended to separate on long term aging. |
| Cellulose (Avicel as above), an acrylic polymer thickener, (known commercially as Permazorb), Quaternium 41 and zinc pyrithione | Lumpy. |
| Hydroxypropylmethylcellulose (know commercially as Methocel), Quaternium 41 and zinc pyrithione. | Stable, but difficult to formulate. Requires heating the water to $176^{\circ}$F., then dispersing the Methocel, then cooling the water until it dissolves and then adding other ingredients. Also, the product tended to separate with long term aging. |
| Quaternium 19 as the suspension agent, Quaternium 41 and zinc pyrithione | The mixture was not compatible, with severe flocculation resulting. |
| Xanthan gum, hydroxyethyl-cellulose, Quaternium 41 and zinc pyrithione | Noticeable incompatibility of the ingredients and poor stability. |
| Hydroxypropylmethylcellulose, hydroxyethylmethylcellulose, Quaternium 41 and zinc pyrithione | The material was hard to work with and had poor stability. |

| Hydroxyethylcellulose alone as the suspension agent, Quaternium 41 and zinc pyrithione | Smooth material, but poor stability. |
| Locust bean gum, Hydroxyethylcellulose, Quaternium 41 and zinc pyrithione | Some difficulty in hydrating the product, and the product had marginal stability. Tends to lump. |
| Guar gum used alone as the suspension agent, Quaternium 41 and zinc pyrithione | Does operate to bring the composition into suspension, but is not satisfactorily asethetically pleasing. |
| Glucan gum used as the suspending agent alone, Quaternium 41 and zinc pyrithione | Does operate to bring the composition into suspension, but is not satisfactorily aesthetically pleasing. |
| Guar gum and hydroxypropylmethylcellulose, Quaternium 41 and zinc pyrithione | Not acceptable, separates on aging. |
| Sodium carboxymethylcellulose, Quaternium 41 and zinc pyrithione | Ingredients not compatible. |

In tests related to those set forth in Table II, Avicel and Quaternium 41 were tested without zinc pyrithione, and Methocel and Avicel were tested with zinc pyrithione, but without Quaternium 41. The Avicel and Quaternium 41 were found incompatible. The Methocel and Avicel and zinc pyrithione without Quaternium 41 gave inconsistent results, with the product tending to be unstable, or at least tending to separate on long term aging.

## TABLE III

### OTHER CATIONIC POLYMERS
### AND OTHER SUSPENSION AGENTS

| MIXTURE | RESULT |
|---|---|
| Magnesium aluminium silicate thickener (sold as Veegum), Quaternium 19 and zinc pyrithione | Flocculation. |
| Magnesium aluminium silicate thickener (sold as Veegum) hydroxypropyl cellulose thickener (sold as Klucel), Quaternium 19 and zinc pyrithione | Flocculation. |
| Cellulose thickener (Avicel GLG11), a cationic cellulosic polymer (sold as Polymer LR), and zinc pyrithione | Incompatible. |
| A cationic acrylic polymer (sold as Reten Spx), cellulose thickener (Avicel GLG11), and zinc pyrithione | Flocculation. |
| Magnesium aluminium silicate (Veegum), Quaternium 40 and zinc pyrithione | Separation in four days at ambient conditions. |
| Magnesium aluminium silicate (Veegum), Quaternium 40, zinc pyrithione and hydroxypropyl cellulose (Klucel) | Lumpy. |
| Magnesium aluminium silicate (Veegum), hydroxypropyl methylcellulose (Methocel), Polyquaternium ammonium chloride, (a cationic polymer sold as Mirapol A-15) and zinc pyrithione | Flocculation. |
| Cellulose thickener (Avicel GLG11), polyquaternium ammonium chloride, a cationic polymer (sold as Mirapol A-15) and zinc pyrithione | Not compatible. |
| Polyquaternium-3, (a cationic polymer sold as Catamer Q), cellulose thickener (Avicel GLG11), zinc pyrithione | Granular and inconsistent, with long term aging being a problem and tending to result in separation. |

| | |
|---|---|
| Polyquaternium ammonium chloride, (a cationic polymer sold as Mirapol A-15) an acrylic polymer thickener (sold as Permazorb), and zinc pyrithione | Not compatible. |
| Magnesium silicate thickener (sold as Laponite XLS) and Quaternium 22 (a cationic polymer sold as Ceraphyl 60) | These were incompatible even before the zinc pyrithione was added. |
| Magnesium aluminium silicate thickener, hydroxypropyl methylcellulose thickener, Quaternium 22 cationic polymer and zinc pyrithione | Flocculation. |
| Magnesium aluminium silicate thickener, hydroxypropyl methylcellulose thickener and polyquaternium ammonium chloride (Mirapol A-15) | Separation occured even before the zinc pyrithione was added. |
| Propylene glycol alginate thickener (sold as Kelecoid HVF), polyquaternium-3 and zinc pyrithione | Separates. |
| Cellulose thickener (Avicel GLG11), Quaternium 22, Quaternium 26 (sold as Cerophyl 65) and zinc pyrithione | Separates. |
| Hydroxypropyl methycellulose thickener, Polyquaternium-3 cationic polymer, Quaternium 22 cationic polymer and zinc pyrithione | Separation |
| Hydroxypropyl methylcellulose thickener, Polyquaternium-3 and zinc pyrithione | Separates |

Other tests were conducted in conjunction with this invention, but tended to focus on combinations of suspending agents and zinc pyrithiones alone, without cationic polymers or alternatively on combinations of suspending agents with cationic polymers.

None of the results of these further tests are considered sufficiently pertinent to report herein.

It is believed that the criticality of the suspension system in accordance with the present invention, for effecting a cream rinse conditioner including zinc pyrithione and a cationic polymer is illustrated by the above test results. In a narrower aspect of the invention, the superiority of Quaternium 41 in a zinc pyrithione anti-dandruff rinse conditioner is also surprisingly illustrated, even though Quaternium 41 cannot be satisfactorily formulated in an anti-dandruff shampoo formulation using zinc pyrithione. Of course it is understood that alternative cationic polymers can be utilized in a commercially acceptable product in accordance with broader aspects of the invention.

It will also be appreciated that various substitutions and modifications can be made without departing from the invention. Also it will be appreciated that while the term "rinse conditioner" is used herein in describing this invention, it is understood that rinse conditioners are called by many names in the marketplace. Our invention may be classified as a rinse conditioner, a conditioning rinse, a cream rinse, a rinse, a conditioner, a cream rinse conditioner, a creme rinse, a creme rinse conditioner, etc.

## CLAIMS

1.    A water base anti-dandruff cream rinse conditioner comprising zinc pyrithione at least in sufficient quantity to provide an effective anti-dandruff agent and a cationic polymer, characterised by sufficient combination of (1) glucan gum, guar gum or mixtures thereof and (2) hydroxyethylcellulose to hold the zinc pyrithione in suspension.

2.    A rinse conditioner as claimed in Claim 1 in which the ingredients (1) and (2) have a proportional relationship of from 3:1 to 1:3 by weight with respect to one another.

3.    A rinse conditioner as claimed in Claim 2 in which the ingredients (1) and (2) are present in approximately equal proportions by weight with respect to one another.

4.    A rinse conditioner as claimed in Claim 1 or Claim 2 or Claim 3 which includes from .25 to 5% by weight active zinc pyrithione; from .25 to 5% of the said cationic polymer; and from .5 to 4% by weight of the said combination of ingredients (1) and (2).

5.    A rinse conditioner as claimed in Claim 4 which includes: about 1% by weight active zinc pyrithione; about 1% by weight of the said cationic polymer; and about 1% by weight of the said combination of ingredients (1) and (2).

6.    A rinse conditioner as claimed in any of
the preceding claims in which the said cationic polymer
is an acrylamide copolymer of dimethyldiallyl ammonium
chloride.

7.    A rinse conditioner as claimed in Claim 6
in which the said cationic acrylamide copolymer of
dimethyldiallyl ammonium chloride has a molecular
weight of approximately 500,000.

8.    A rinse conditioner as claimed in Claim 6
or Claim 7 in which the cationic polymer is selected
from:  Quaternium 41; Quaternium 19; Quaternium 22;
Quaternium 23; Quaternium 26; Quaternium 40;
Polyquaternium 3; Quaternium 33; Quaternium 31 and
combinations of two or more of the aforesaid cationic
polymers.

9.    A rinse conditioner as claimed in any of
the preceding claims which includes additional
conditioning ingredients.

10.    A rinse conditioner as claimed in Claim 9 in
which the additional conditioning ingredients are
present in an amount of up to 18%.

11:    A rinse conditioner as claimed in Claim 10
in which the amount by weight of the additional conditioning ingredients is approximately 9%.

12.   A rinse conditioner as claimed in Claim 9 or Claim 10 or Claim 11 in which the additional conditioning ingredients are selected from:   a mixture of stearyl alcohol and cetrimonium bromide; steartrimonium hydrolyzed animal protein; isostearamido propyl morpholine lactate; stearalkonium chloride; tallow amidoethyl polyhydroxy ether ammonium chloride; hydrogenated tallow poly oxyethylene ammonium ethosulphate; cetrimonium chloride; dialkyl dimethyl ammonium chloride; other quaternary ammonium salts; and combinations of two or more of the aforesaid ingredients.

13.   A rinse conditioner as claimed in Claim 9 in which the additional conditioning ingredients include at least a combination of stearyl alcohol and cetrimonium bromide in an amount of from 1 to 5% by weight.

14.   A rinse conditioner as claimed in Claim 13 in which the said combination of stearyl alcohol and cetrimonium bromide is present in an amount of about 3% by weight.

15.   A water base, anti-dandruff rinse conditioner characterised by the use of the combination of an effective amount of zinc pyrithione and a cationic acrylamide copolymer of dimethyldiallyl ammonium chloride having a molecular weight of approximately 500,000.

16.   A rinse conditioner as claimed in Claim 15
which includes additional conditioning ingredients.

17.   A rinse conditioner as claimed in Claim 16
in which the additional conditioning ingredients are
present in an amount of up to about 18%.

18.   A rinse conditioner as claimed in Claim 17
in which the amount by weight of the additional
conditioning ingredients is approximately 9%.

19.   A rinse conditioner as claimed in Claim 16
or Claim 17 or Claim 18 which additionally includes
a suspension system comprising a combination of (1) one
of glucan gum, guar gum or mixtures thereof, and
(2) hydroxyethylcellulose, in proportions to one another
of from about 3:1 to about 1:3 parts by weight.

20.   A rinse conditioner as claimed in Claim 19
in which the suspension system is present in an amount
of from .5 to 4% by weight of the conditioner.